Europäisches Patentamt

European Patent Office

Office européen des brevets

(11) Publication number: **0 462 795 A2**

# EUROPEAN PATENT APPLICATION

(21) Application number: 91305496.1

(22) Date of filing: 18.06.91

(51) Int. Cl.⁵: **C07H 1/00**, C07H 1/06, C08B 37/00, // G01N35/00

(30) Priority: 21.06.90 GB 9013828
21.06.90 GB 9013830
21.06.90 GB 9013831

(43) Date of publication of application:
27.12.91 Bulletin 91/52

(84) Designated Contracting States:
DE ES FR GB IT

(71) Applicant: OXFORD GLYCOSYSTEMS LIMITED
Unit 4, Hitching Court, Blacklands Way
Abingdon, Oxon, OX14 1RG (GB)

(72) Inventor: Parekh, Rajesh Bhikhu
2 Pound Close
Kirtlington Oxon OX5 3JR (GB)
Inventor: Merry, Anthony Hugh
22 Little Lees
Charlbury Oxon OX7 3HB (GB)
Inventor: Bruce, James
53 Wytham View
Eynsham Oxon OX8 1LY (GB)

(74) Representative: Courtney, Graham Sidney
Whitegates
East Hanney Wantage Oxon OX12 0HR (GB)

(54) Isolation of glycans.

(57) The present invention relates to a method for isolating glycans.
The invention provides a method for isolating a glycan which has been released from a glycoconjugate which method includes sorbing the glycan (and any derivative thereof) upon a chromatographic material, separating the glycan from any remaining conjugate and/or conjugate-derived material, and recovering the glycan.

EP 0 462 795 A2

Jouve, 18, rue Saint-Denis, 75001 PARIS

FIG. 1

The present invention relates to a method for isolating glycans.

According to one aspect of the present invention there is provided a method for isolating a glycan (as hereinafter defined) which has been released from a glycoconjugate which method includes sorbing the glycan (and any derivative thereof) upon a chromatographic material, separating the glycan from any remaining conjugate and/or conjugate-derived material, and recovering the glycan.

The sorbing of the glycan upon a chromatographic material and suitable elution of the chromatographic material may be used to separate glycan from conjugate and/or conjugate-derived material. The separation of glycan from any remaining conjugate and/or conjugate-derived material may be effected, for example, by further chromatography.

The glycan may be released from the glycoconjugate in any convenient manner, for example by use of a releasing agent as hereinafter disclosed.

According to one embodiment of the present invention there is provided a method for isolating a glycan (as hereinafter defined) from a post-release reaction mixture formed by the release of the glycan from a glycoconjugate which method includes contacting the post-release reaction mixture with a chromatographic material thereby to effect sorbing of the glycan (and any derivative thereof) upon the chromatographic material, eluting the chromatographic material, said sorbing and said eluting being such as to effect separation of glycan (and any derivative thereof) from conjugate and/or conjugate-derived material and such as to remove any releasing agent used in the release of glycan from the glycoconjugate, separating the glycan from any remaining conjugate or conjugate-derived material, and recovering the glycan.

The glycan may be separated from any remaining conjugate and/or conjugate-derived material in any suitable manner. Thus, for example, the glycan and remaining conjugate and/or conjugate-derived material may be eluted from the chromatographic material and subjected to further separation (e.g. by means of chromatography).

As used in this Specification the term "glycan" embraces N-glycan, O-glycan, and N- and O-glycans.

It will be appreciated that an N-glycan is an oligosaccharide which is covalently bonded to a conjugate by an N-glycosidic linkage (i.e. an N-linked type oligosaccharide) and that an O-glycan is an oligosaccharide which is covalently bonded to a conjugate by an O-glycosidic linkage (i.e. an O-linked type oligosaccharide).

Also it will be appreciated that N-glycans and/or O-glycans occur in glycoconjugates. In such glycoconjugates, glycans are covalently bonded (via an N-glycosidic linkage in the case of an N-glycan or an O-glycosidic linkage in the case of an O-glycan) to a conjugate (e.g. a peptide conjugate).

Examples of glycoconjugates are glycoproteins, glycohormones, glycolipids and mucins.

A releasing agent may be any agent which gives rise to cleavage of an N-glycosidic bond and/or an O-glycosidic bond such as to release an N-glycan and/or an O-glycan from the conjugate of a glycoconjugate.

For example, a post-release reaction mixture for treatment in accordance with the present invention may be formed by reacting together a glycoconjugate and a releasing agent.

Examples of releasing agents are enzymes and chemical reagents (e.g. an alkaline solution or a hydrazine reagent (such as substantially anhydrous hydrazine)).

An enzyme may be a non-derivatising releasing agent (i.e. a releasing agent which does not cause derivatisation of the glycan after it has been released from the conjugate).

Where, by way of example, a glycan has been released from a glycoconjugate by use of a derivatising releasing agent (i.e. one which causes derivatisation of a glycan after its release from the conjugate) it may be desirable to perform a reaction to regenerate unreduced glycan.

For example, a hydrazine reagent may give rise to glycan derivatives having free primary amino acid groups and thus an N-acetylation reaction may be performed to generate unreduced glycan.

Also , by way of example, if the form of N-acetylation reaction used introduces unwanted mono- and/or di-valent cations these may be removed prior to separating the glycan from any remaining conjugate and/or conjugate derived material and recovering the glycan.

Also, if any derivatives of the glycan are present prior to recovering the glycan, these derivatives may be converted to unreduced glycan.

Thus, in a further embodiment the present invention provides a method for isolating a glycan (as hereinbefore defined) from a post-release reaction mixture formed by the release of the glycan from a glycoconjugate which method includes contacting the post-release mixture with a first chromatographic material thereby to effect sorbing of the glycan (and any derivative thereof) upon the chromatographic material, eluting the chromatographic material, said sorbing and said eluting being such as to effect separation of glycan (and any derivative thereof) from conjugate and/or conjugate-derived material and such as to remove any releasing agent used in the release of glycan from the glycoconjugate, and, if a derivative of a glycan is present which has free primary amino acid groups, performing an N-acetylation reaction to generate unreduced glycan from any glycan derivative which has free primary amino acid groups, removing mono- and/or di-valent cations from a reaction mixture obtained after the N-acetylation reaction, if the manner in which the N-acetyl reaction was performed makes this desirable, separating from the reaction mixture glycan, or a derivative thereof, by means of sorption upon a second chromatographic material and selective elution therefrom, and converting any derivative of the glycan to unreduced glycan.

By way of example, a glycan and any derivative thereof may be eluted from the first chromatographic material prior to performing an N-acetylation reaction.

Alternatively, by way of example, a glycan and any derivative thereof may be subjected to an N-acetyl reaction prior to being eluted from the first chromatographic material.

Further, by way of example, where an acetohydrazone derivative of the glycan is present (e.g. after elution from the second chromatographic material) acidic conditions may be used to effect generation of unreduced glycan. Thus, for example, mildly acidic conditions may be applied by means of acid in immobilised form (i.e. immobilised acid species), mineral acid or Lewis acid.

Where the releasing agent is a hydrazine reagent, the hydrazine reagent may be, for example, hydrazine itself or any suitable derivative or compound thereof (e.g. related salt) capable of bringing about a desired cleavage of an N-glycosidic and/or an O-glycosidic bond linking an N-glycan and/or an O-glycan to a conjugate. The hydrazine reagent may be used, for example, in liquid form (e.g. in anhydrous form) or in vapour form.

The release and subsequent isolation of N-glycans and/or O-glycans from a glycoconjugate is important for several reasons. Principal among these are the following: firstly, a detailed structural characterisation of a glycoconjugate requires structural analysis of any associated glycans. Since several such glycans may be attached to a single conjugate, and since structural analysis of glycans is most accurately performed on single, purified glycans, such an analysis requires prior release of such glycans from the conjugate and subsequent isolation of such glycans from the conjugate, and then purification of glycans from one another; secondly, such glycans are increasingly recognised as important biological molecules in their own right. A study of the biological properties of N-glycans and O-glycans is preferentially undertaken using N-glycans and O-glycans free of the conjugate.

By way of example the present invention has been applied to the isolation of N-glycans, O-glycans, and N- and O-glycans following the release of such glycans from glycoconjugates using a hydrazine reagent. Thus, in accordance with the present invention, N-glycans have been isolated after release from bovine fetuin, human serum $\alpha$1-acid glycoprotein, horseradish peroxidase and hen egg ovalbumin; O-glycans have been isolated after release from bovine fetuin, hog sub-maxillary mucin, and asialo globoside; and N- and O-glycans have been isolated after release from bovine fetuin.

Examples of chromatographic materials which may be used in accordance with the present invention are microcrystalline celluloses and $C_{18}$ reverse-phase materials.

It will be appreciated that, in accordance with the present invention, isolation may, for example, be used to isolate glycans from conjugate in a manner independent of the nature of both the glycan (i.e. oligosaccharide component) and the conjugate.

Also, for example, isolation in accordance with the present invention may be achieved without causing irreversible chemical damage to the released glycans.

By way of example, a high yield of released glycan may be obtained in accordance with the present invention.

A high yield may be defined as $\geq 85\%$, but may be less than this as long as the yield is useful in practice.

Thus, although a preferred yield is a high yield such as $\geq 85\%$ there may be applications where lower yields are acceptable.

It will be appreciated that following separation of any releasing agent and removal of conjugate and/or conjugate-derived material from the released glycan, glycan may be obtained, for example, as an unfractionated pool free of releasing agents and conjugate or conjugate-derived material.

It is to be understood that a method for isolating a glycan in accordance with the present invention may be independent of the way in which the glycan was released from the glycoconjugate. The method of the present invention may be applied to the isolation of released glycan irrespectively of how the glycan was released from a glycoconjugate (e.g. chemically or enzymatically) and irrespectively of whether, or not, the glycan isolation involves unreduced glycan or a derivative of a glycan.

Also, a method of isolation in accordance with the invention does not necessarily require a knowledge of how a post-release reaction mixture was formed since all of the steps (e.g. separation, N-acetylation and acid treatment) may be carried out on a post-release reaction mixture irrespectively of how the post-reaction mixture was formed. (It will be appreciated that in some cases a step of the method of the present invention may be redundant (e.g. the N-acetylation reaction if no derivatives are present having free primary amino acid groups or the acid treatment if no acetohydrazone derivative is present). However, since all of the steps may be carried out on a post-release reaction mixture a decision need not necessarily be made as to which steps need to be carried out in a particular case. Thus, the same method of isolation in accordance with the present invention may be applied irrespectively of the post-release reaction mixture.)

The present invention will now be further described, by way of example only, with reference to the Figures of the accompanying Drawings and with reference to Examples 1 to 7.

Referring now to the drawings:

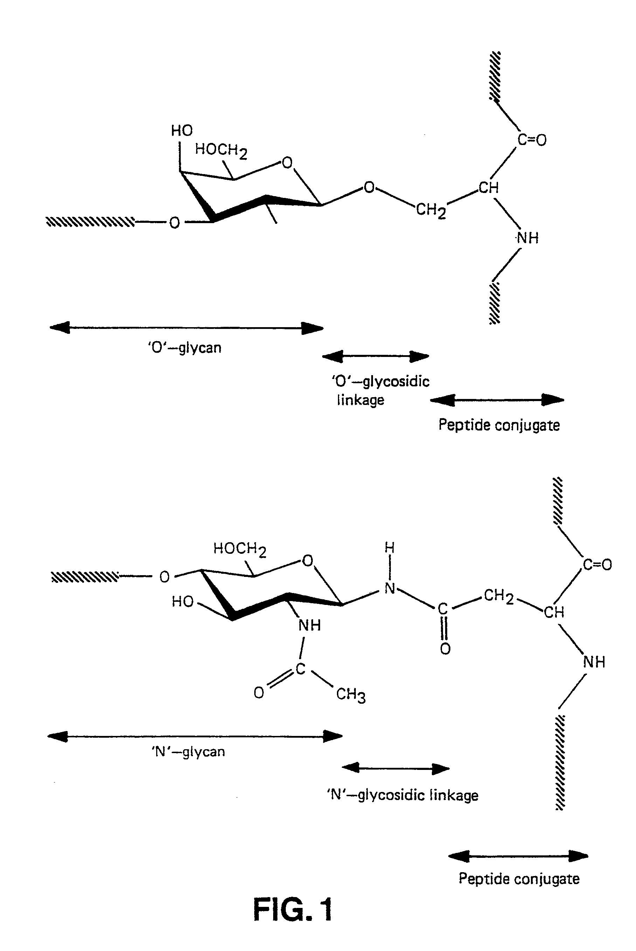

Figure 1 shows representations of an O-glycosidic linkage and an N-glycosidic linkage attaching an N-glycan and an O-glycan to a peptide conjugate;

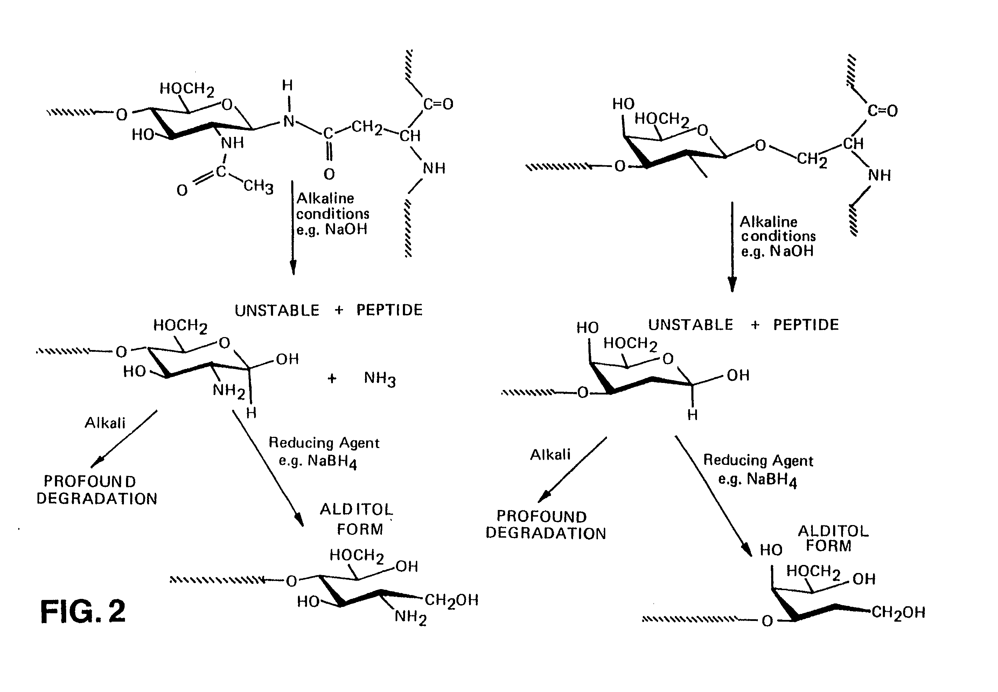

Figure 2 shows a summary of significant chemical reactions in currently practised alkaline cleavage of N-glycosidic and O-glycosidic linkages;

Figure 3 is a high voltage radioelectrophoretogram of radiolabelled glycan (isolated in accordance with Example 1) after deacidification;

Figure 4 is a gel filtration chromatogram of radiolabelled glycans (glycans isolated in accordance with Example 1);

Figure 5 is a size chromatogram of glycan alditols derived from bovine serum fetuin (see Example 2);

Figure 6 is a size chromatogram of glycan alditols derived from human serum $\alpha$1-acid glycoprotein (see Example 3);

Figure 7 is a size chromatogram of alditols derived from horseradish peroxidase (Example 4);

Figure 8 is a size chromatogram of alditols derived from hen egg ovalbumin (Example 5);

Figure 9 is a high-voltage radioelectrophoretogram of the radiolabelled glycan (charge chromatogram of glycan alditols derived from bovine serum fetuin) (Example 6);

Figure 10 is a gel filtration chromatogram of the radiolabelled glycans after deacidification (size chromatogram of glycan alditols derived from bovine serum fetuin) (Example 6);

Figure 11 is a charge chromatogram of glycan alditols derived from hog sub-maxillary mucin (Example 6) ;

Figure 12 is a size chromatogram of glycan alditols derived from hog sub-maxillary mucin (Example 7); and

Figure 13 is a size chromatogram of glycan alditols derived from asialo globoside (Example 8).

*Example 1: Isolation of N- and O-glycans from Bovine Serum Fetuin*

In this example, N- and O-glycans were isolated from the glycoprotein bovine fetuin and the N- and O-glycans so recovered assessed with respect to integrity, yield and purity.

Fetuin from foetal calf serum was purchased from the Sigma Chemical Company, (Poole, Dorset, UK). 1 milligramme of the glycoprotein was rendered salt-free by exhaustive microflow dialysis against glass-distilled water. After dialysis, the solution was lyophilised in a clean glass reaction tube using an Edwards Modulyo freeze-drier operating at 25 millibar vacuum. To the lyophilised glycoprotein was added 0.5ml of anydrous hydrazine (of relative water content ~2.0% v/v). The tube was sealed under an anhydrous and oxygen-free atmosphere at room temperature and then placed in an oven equilibrated at 95°C for 3.75 hours.

After this time, unreacted hydrazine was removed from the reaction mixture in the tube or by column chromatography as follows. To the top of a pre-prepared, pre-equilibrated (in butanol, ethanol, acetic acid solvent of composition 4:1:0.15, v/v/v) column (bed volume 2ml) of microcrystalline cellulose (Avicel®, was added 4ml of equilibration solvent without flow. The reaction mixture from the tube (i.e. hydrazinolysate) (0.5ml) was then added to the column, followed by thorough mixing to create a single-phase, all without flow. Liquid flow was then begun, and when all liquid had passed through the column, the column was washed with 3 column volumes of equilibration solvent. The column was next washed with 1 column volume of methanol, and then two column volumes of aqueous sodium acetate (200 mM, pH 5.0). Re-N-acetylation was performed by the addition of 0.5ml acetic anhydride to pooled elutant and incubation at room temperature for 1 hour.

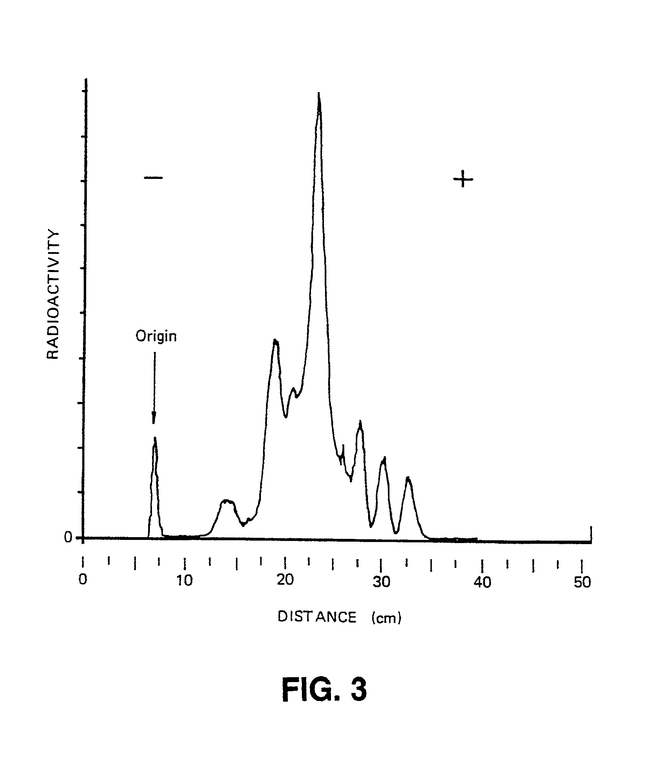

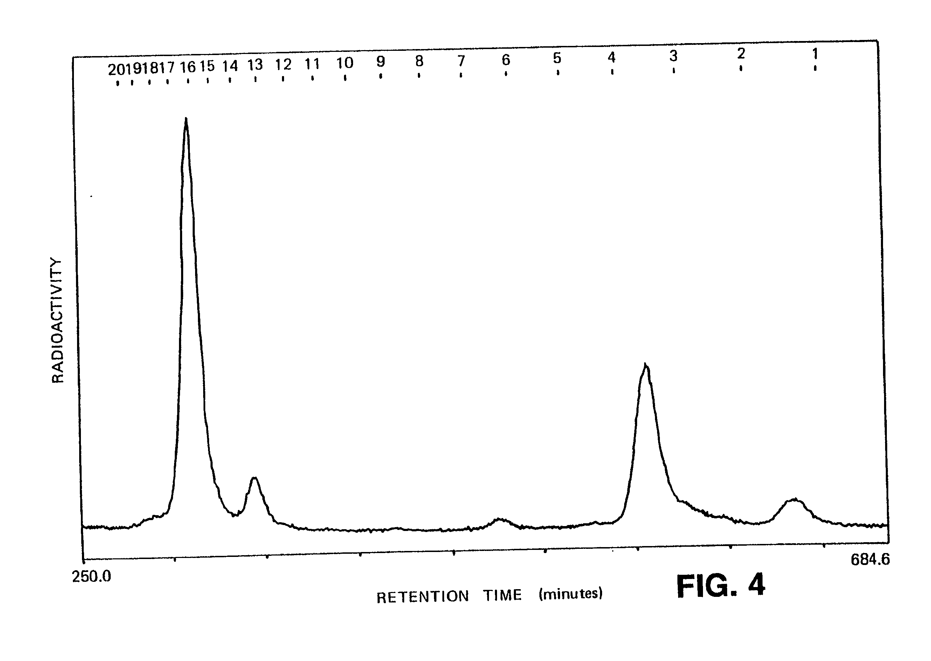

The resulting N-acetylation mixture was then passed through a Dowex AG50X12 (H$^+$) column of volume 2.2ml, and the column was then washed with five column volumes of distilled water. The elutant and washings were combined and then rotary evaporated to dryness. The entire sample was then taken up in 0.3ml glass distilled water and applied to the top of a column of pre-prepared, pre-equilibrated microcrystalline cellulose (0.8cm x 5cm) containing 2.0ml of butanol, methanol (4:1, v/v) above the cellulose bed, without flow. The aqueous phase and the butanol/ethanol phase were mixed thoroughly into a single homogeneous phase, and flow was then begun. After all the liquid had passed through, the column was washed with 5 column volumes of butanol, ethanol, water solvent (of composition 4:1:0.5, v/v/v). The column was next washed with 1 column volume of methanol and 2 column volumes of distilled water. The methanol and water fractions were co-pooled, rotary evaporated, taken up in 0.2ml of 1mM aqueous copper (II) acetate solution, incubated at room temperature fo 30 minutes, and passed through a tandem column of Chelex 100 (Na$^+$) and Dowex AG50X12 (H$^+$) containing 300µl of each resin. The column was washed with five column volumes of water and the eluant and washings co-pooled, filtered (0.2μM Teflon membrane filter), rotary evaporated to dryness, and taken up in 1ml of glass distilled water. Aliquots of the unreduced oligosaccharide fraction were analysed for monosaccharide composition and amino acid content by the ninhydrin method. (The results are given in Table I below.) An aliquot of the unreduced oligosaccharides was radiolabelled by reduction in alkaline $NaB^3H_4$ and the radiolabelled oligosaccharide alditols so obtained were analysed with respect to charge and size (after deacidification). The results are given in Figures 3 and 4 of the accompanying Drawings. The overall conclusion of these analyses is that contaminants other than peptide material are not detectable, that the level of peptide is <10% (by weight), and that glycans are intact, and recovered in high (e.g. $\geqq$85%) yield.

## Table I

|  | Total content of N-acetyl-galactosamine * (nanomoles) | Total content of N-acetyl-glucosamine* (nanomoles) | Total content of amino acids (nanomoles) |
|---|---|---|---|
| Starting Glycoprotein | 52.5 | 252.5 | 6918 |
| Final glycan pool | 45.1 | 217.2 | 332 |

*In the glycoprotein fetuin it is generally accepted that the content of N- and O-glycans is directly related to the content of N-acetylglucosamine and N-acetylgalacto-samine, respectively. The contents of N-acety-lglucosamine and N-acetylgalactosamine are therefore used as a preferred measure of the N- and O-glycan content in both the fetuin glycoprotein and the glycan pool obtained after performing the methods described in this invention.

By treating the glycoprotein bovine fetuin in accordance with the present invention, it has been established that certain sequences of processing events allow recovery with high yield (e.g.$\geqq$85%), of intact N- and O-glycans free of contaminants. A sequence of events has been defined by which intact N- and O-glycans can be so recovered, and this sequence is such as to be readily automated. That this is so is proven by the construction and successful functioning of a machine which is able to receive a lyophilised sample of glycoconjugate and successfully deliver the intact N- and O-glycans previously associated with that glycoconjugate.

### Example 2: Isolation of N-glycans from Bovine Fetuin

In this Example N-glycans were isolated from the glycoprotein bovine fetuin and the N-glycans so recovered assessed with respect to integrity, yield and purity.

Fetuin from foetal calf serum was purchased from the Sigma Chemical Company, (Poole, Dorset, UK). 1 milligramme of the glycoprotein was rendered salt-free by exhaustive microflow dialysis against glass-distilled water. After dialysis, the solution was lyophilised in a clean glass reaction tube using an Edwards Modulyo freeze-drier operating at 25 millibar vacuum. To the lyophilised protein was added 0.5ml of anydrous hydrazine (of relative water content ~2.0% v/v). The tube was sealed under an anhydrous and oxygen-free atmosphere at room temperature and then placed in an oven equilibrated at 100°C for 10 hours.

After this time, unreacted hydrazine was removed from the reaction mixture in the tube by column chromatography as follows: to the top of a pre-prepared, pre-equilibrated (in butanol, ethanol, acetic acid solvent of composition 4:1:0.15, v/v/v) column of microcrystalline cellulose (Avicel®), was added 4ml of equilibration solvent without flow. The reaction mixture from the tube (i.e. the hydrazinolysate) (0.5ml) was then added to the solvent, followed by thorough mixing to create a single-phase, all without flow. Liquid flow was then begun, and when all liquid had passed through the column, the column was washed with 3 column volumes of equilibration solvent. The column was next washed with 1 column volume of methanol, and then two column volumes of aqueous sodium acetate (200 mM, pH 5.0). Re-N-acetylation was performed by the addition of 0.5ml acetic anhydride to pooled elutant and incubation at room temperature for 1 hour.

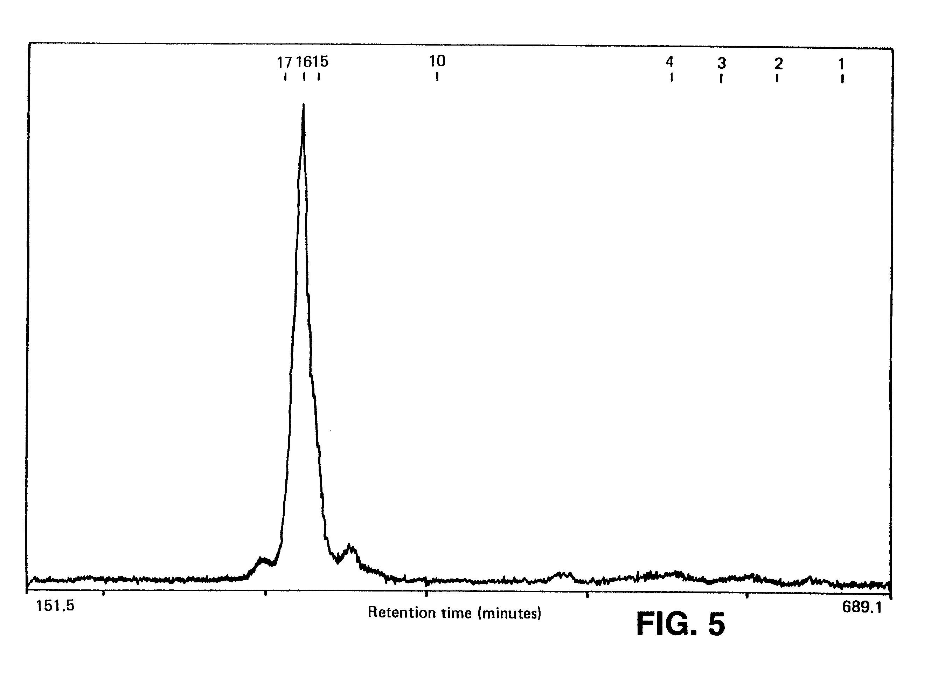

The resulting N-acetylation mixture was then passed through a Dowex AG50X12 (H⁺) column of volume 2.2ml, and the column was then washed with five column volumes of distilled water. The elutant and washings were combined and then rotary evaporated to dryness. The entire sample was then taken up in 0.3ml glass distilled water and applied to the top of a column of pre-prepared, pre-equilibrated microcrystalline cellulose (0.8cm x 5cm) containing 2.0ml of butanol, methanol (4:1, v/v) above the cellulose bed without flow. The aqueous phase and the butanol/ethanol phase were mixed thoroughly into a single homogeneous phase, and flow was then begun. After all the liquid had passed through, the column was washed with 5 column volumes of butanol, ethanol, water solvent (of composition 4:1:0.5, v/v/v). The column was next washed with 1 column volume of methanol and 2 column volumes of distilled water. The methanol and water fractions were co-pooled, rotary evaporated, taken up in 0.2ml of 1mM aqueous copper (II) acetate solution, incubated at room temperature for 30 minutes, and passed through a tandem column of Chelex 100 (Na⁺) and Dowex AG50X12 (H⁺) containing 300μl of each resin. The column was washed with five column volumes of water and the eluant and washings co-pooled, filtered (0.2μM Teflon membrane filter), rotary evaporated to dryness, and taken up in 1ml of glass distilled water. An aliquot of the unreduced oligosaccharides was analysed for monosaccharide composition and amino acid content by the ninhydrin method. (The results are given in Table II below.) Another aliquot of the unreduced oligosaccharides was radiolabelled by reduction in alkaline $NaB^3H_4$ and the radiolabelled oligosaccharide alditols so obtained were deacidified and analysed with respect to size. (The results are given in Figure 5 of the accompanying drawings.)

*Example 3: Isolation of N-glycans from human Serum α1-acid glycoprotein*

In this Example essentially the same procedure was followed as given in Example 2 with the exception that the glycoconjugate was human serum α1-acid glycoprotein not bovine fetuin.

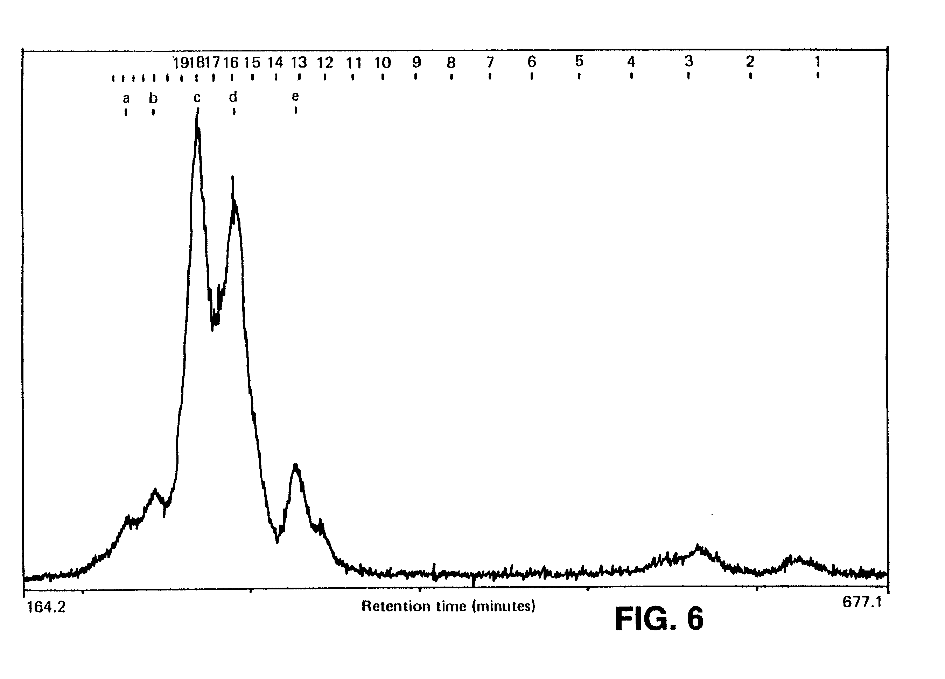

Results of analyses for monosaccharide composition and amino acid content are given in Table II below. The results of size analysis are given in Figure 6 of the accompanying Drawings.

*Example 4: Isolation of N-glycans from Horseradish Peroxidase*

In this Example essentially the same procedure was followed as given in Example 2 with the exception that the glycoconjugate was horseradish peroxidase and not bovine fetuin.

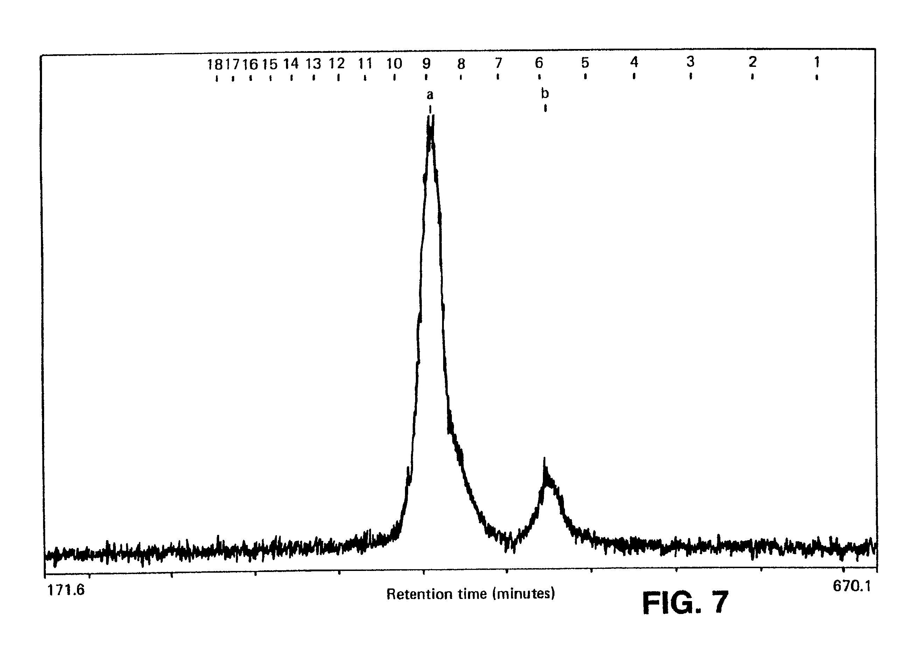

Results of analyses for monosaccharide composition and amino acid content are given in Table II below. The results of size analysis are given in Figure 7 of the accompanying Drawings.

*Example 5: Isolation of N-glycans from Hen Egg Ovalbumin*

In this Example essentially the same procedure was followed as given in Example 2 with the exception that the glycoconjugate was hen egg ovalbumin.

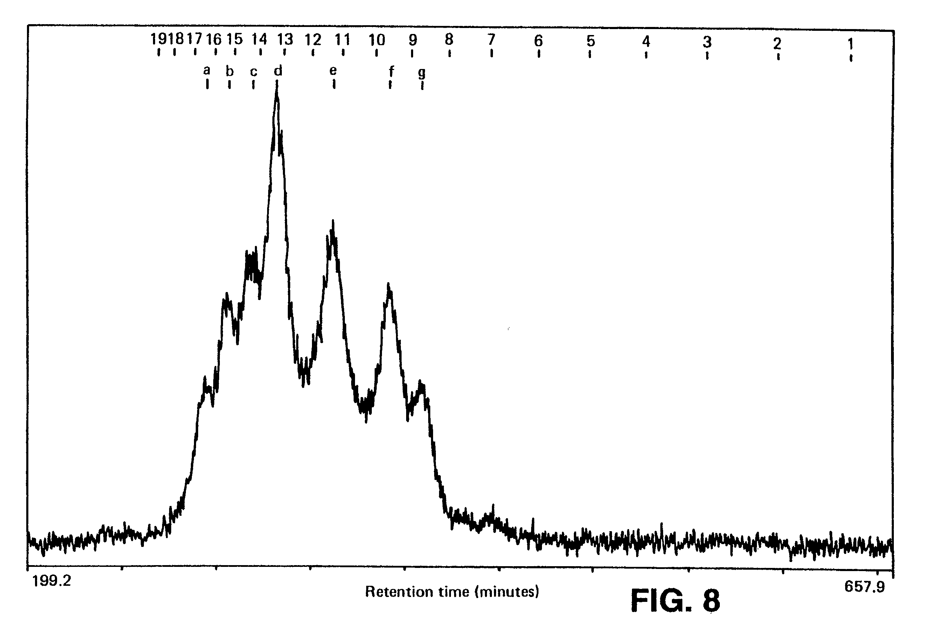

Results of analyses for monosaccharide composition and amino acid content are given in Table II below. The results of size analysis are given in Figure 8 of the accompanying Drawings.

The overall conclusion of these analyses is that contaminants other than peptide material are not detectable, that the level of peptide is <10% (by weight), and that N-glycans are intact, and recovered in high (e.g. ≧85%) yield.

## Table II

| | Total content of N-acetyl-glucosamine (nanomoles)* | Total content of amino acids (µgrams) |
|---|---|---|
| Starting bovine fetuin | 319 | 1000 |
| Bovine fetuin N-glycan pool | 284 | 47 |
| Starting human serum α1-acid | 801 | 1000 |
| Human serum α1-acid N-glycan pool | 746 | 53 |
| Starting horseradish peroxidase | 246 | 1000 |
| Horseradish peroxidase N-glycan pool | 230 | 61 |
| Starting hen egg ovalbumin | 192 | 1000 |
| Hen egg ovalbumin N-glycan pool | 171 | 39 |

*It is generally accepted that the content of N-glycans is directly related to the content of N-acetylglucosamine. The content of N-acetylglucosamine is therefore used as a preferred measure of the N-glycan content in both the starting glycoconjugate and the glycan pool obtained after performing the methods described.

By treating the above glycoconjugates in accordance with the present invention, it has been established that certain sequences of processing events allow recovery with high yield (e.g. ≧85%), of intact N-glycans free of contaminants. A sequence of events has been defined by which intact N-glycans can be so recovered, and this sequence is such as to be readily automated. That this is so is proven by the construction and successful functioning of a machine which is able to receive a lyophilised sample of glycoconjugate and successfully deliver the intact N-glycans previously associated with that glycoconjugate.

*Example 6: Isolation of O-glycans from Bovine Fetuin*

In this example, O-glycans were isolated from the glyco-protein bovine fetuin and the O-glycans so recovered assessed with respect to integrity, yield and purity.

Fetuin from foetal calf serum was purchased from the Sigma Chemical Company, (Poole, Dorset, UK). 1 milligramme of the glyco-protein was rendered salt-free by exhaustive microflow dialysis against glass-distilled water. After dialysis, the solution was lyophilised in a clean glass reaction tube using an Edwards Modulyo freeze-drier operating at 25 millibar vacuum. To the lyophilised glyco-protein was added 0.5ml of anydrous

8

hydrazine (of relative water content ~2.0% v/v). The tube was sealed under an anhydrous and oxygen-free atmosphere at room temperature and then placed in an oven equilibrated at 65°C for 8 hours.

After this time, unreacted hydrazine was removed from reaction mixture in the tube by column chromatography as follows: to the top of a pre-prepared, pre-equilibrated (in butanol, ethanol, acetic acid solvent of composition 4:1:0.15, v/v/v) column (bed volume 2ml) of microcrystalline cellulose (Avicel®), was added 4ml of equilibration solvent without flow. The reaction mixture from the tube (i.e. the hydrazinolysate) (0.5ml) was then added to the column, followed by thorough mixing to create a single-phase, all without flow. Liquid flow was then begun, and when all liquid had passed through the column, the column was washed with 3 column volumes of equilibration solvent. The column was next washed with 1 column volume of methanol, and then two column volumes of aqueous sodium acetate (200mM, pH 5.0). Re-N-acetylation was performed by the addition of 0.5ml acetic anhydride to pooled elutant and incubation at room temperature for 1 hour.

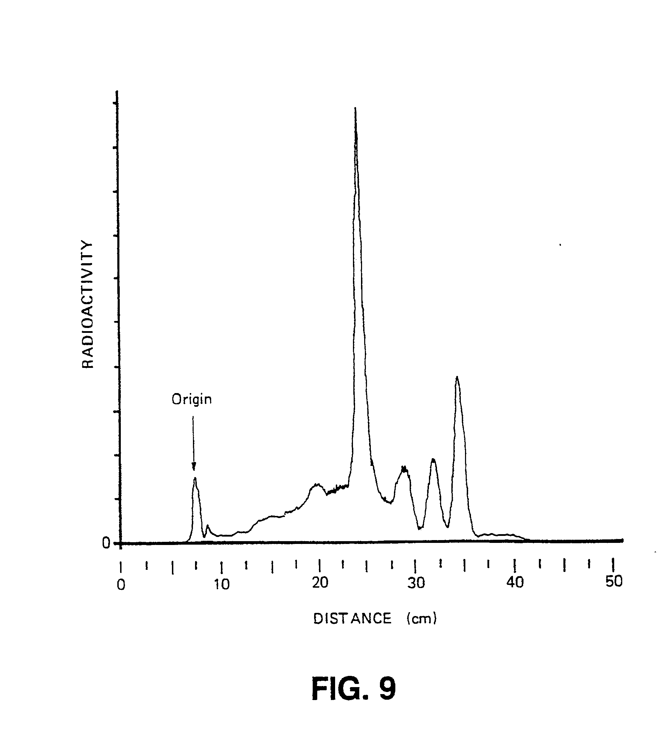

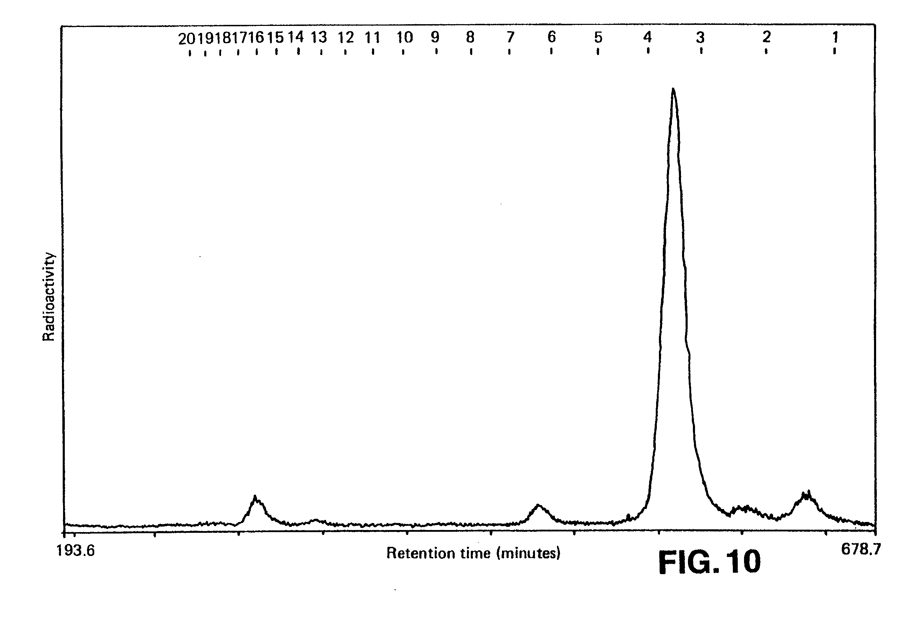

The resulting N-acetylation mixture was then passed through a Dowex AG50X12 (H⁺) column of volume 2.2ml, and the column was then washed with five column volumes of distilled water. The elutant and washings were combined and passed through a $C_{18}$ reverse-phase cartridge (Waters SEP-PAK $C_{18}$ cartridge from the Waters Division of Millipore Corporation, Millford, Mass U.S.A.) previously prepared by washing with 2 volumes of methanol and 5 volumes of water. The $C_{18}$ cartridge was washed with 2 volumes of water and the eluant and washings combined, and then rotary evaporated to dryness. The entire sample was then taken up in 0.3ml glass distilled water and applied to the top of a column of pre-prepared, pre-equilibrated microcrystalline cellulose (0.8cm x 5cm) containing 2.0ml of butanol, methanol (4:1, v/v) above the cellulose bed without flow. The aqueous phase and the butanol/ethanol phase were mixed thoroughly into a single homogeneous phase, and flow was then begun. After all the liquid had passed through, the column was washed with 5 column volumes of butanol, ethanol, water solvent (of composition 4:1:0.5, v/v/v). The column was next washed with 1 column volume of methanol and 2 column volumes of distilled water. The methanol and water fractions were co-pooled, rotary evaporated, taken up in 0.2ml of 1mM aqueous copper (II) acetate solution, incubated at room temperature for 30 minutes, and passed through a tandem column of Chelex 100 (Na⁺) and Dowex AG50X12 (H⁺) containing 300μl of each resin. The column was washed with five column volumes of water and the eluant and washings co-pooled, filtered (0.2μM Teflon membrane filter), rotary evaporated to dryness, and taken up in 1ml of glass distilled water. An aliquot of the unreduced oligosaccharide fraction was analysed for monosaccharide composition and amino acid content by the ninhydrin method. (The results are given in Table III.) An aliquot of the unreduced oligosaccharide fraction was radiolabelled by reduction in alkaline $NaB^3H_4$ and the radiolabelled oligosaccharide alditols so obtained were analysed with respect to charge and size. The results are given in Figures 9 and 10 of the accompanying Drawings.

*Example 7: Isolation of O-glycans from Hog Sub-maxillary Mucin*

In this Example essentially the same procedure was followed as given in Example 6 with the exception that the glycoconjugate was hog sub-maxilliary mucin not bovine fetuin.

Results of analyses for monosaccharide composition and amino acid content are given in Table III below.

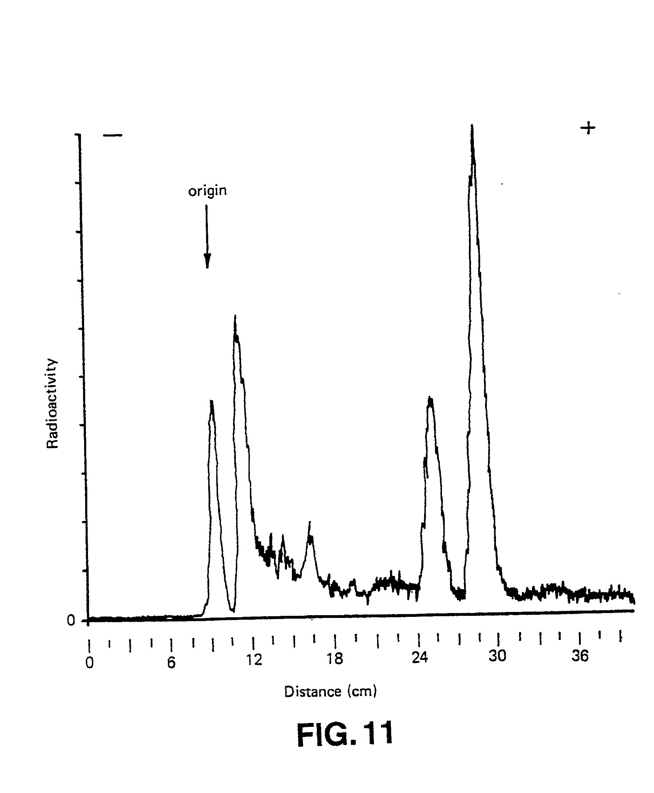

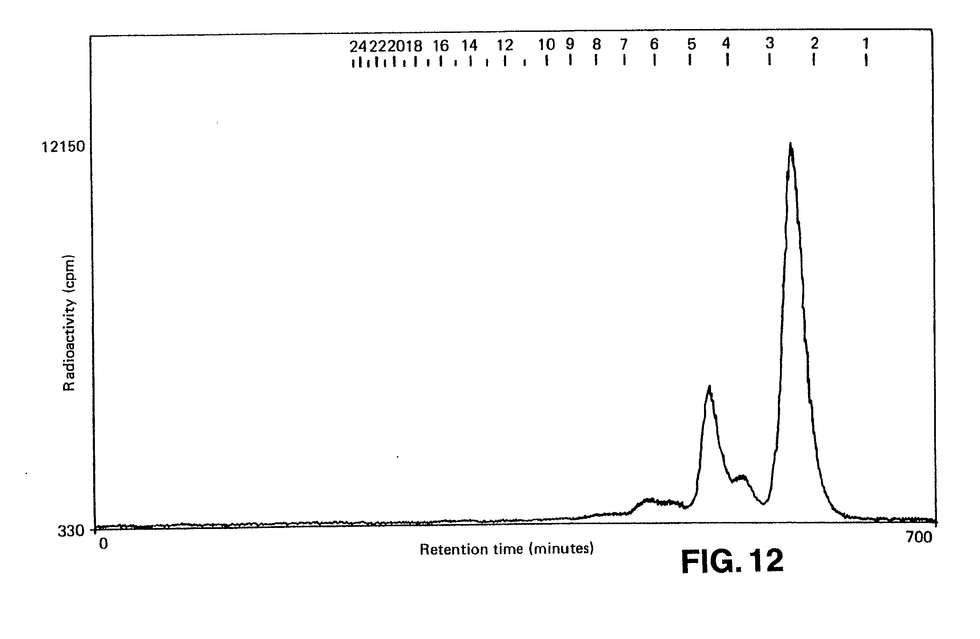

The results of charge and size analyses are given in Figures 11 and 12 of the accompanying Drawings.

*Example 8: Isolation of O-glycans from Asialo Globoside*

In this Example essentially the same procedure was followed as given in Example 6 with the exception that the glycoconjugate was asialo globoside not bovine fetuin.

Results of analyses for monosaccharide composition and amino acid content are given in Table III below.

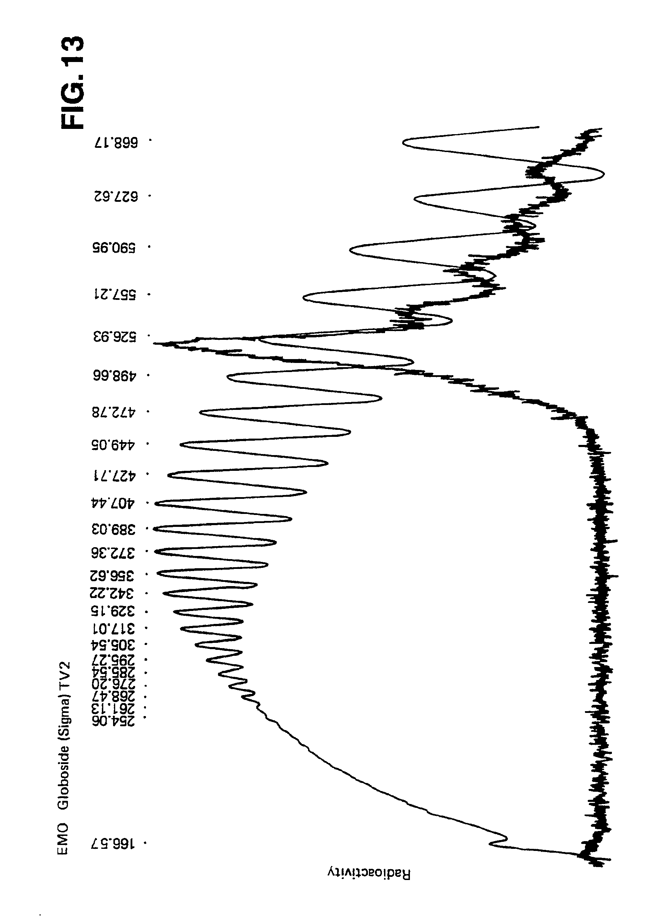

The result of size analysis is given in Figure 13 of the accompanying Drawings.

The overall conclusion of these analyses is that contaminants other than peptide material are not detectable, that the level of peptide is <10% (by weight), and that O-glycans are intact, and recovered in high (e.g. ≧85%) yield.

## Table III

|  | Total content of N-acetyl-galactosamine * (nanomoles) | Total content of amino acids (μgrams) |
|---|---|---|
| Starting bovine fetuin | 69 | 1000 |
| Bovine fetuin O-glycan pool | 60 | 74 |
| Starting hog sub-maxiallary mucin | 603 | 1000 |
| Hog sub-maxiallary mucin O-glycan pool | 561 | 56 |
| Starting Asialo globoside | 963 | 0 |
| Asialo globoside O-glycan pool | 831 | 0 |

*It is generally accepted that the content of O-glycans is directly related to the content of N-acetylgalactosamine. The contents of N-acetylgalactosamine is therefore used as a preferred measure of the O-glycan content in both the starting glycoconjugate and the glycan pool obtained after performing the methods described.

By treating the above glycoconjugates in accordance with the present invention, it has been established that certain sequences of processing events allow recovery with high yield, of intact O-glycans free of contaminants. A sequence of events has been defined by which intact O-glycans can be recovered in high yield and free of contaminants, and this sequence is such as to be readily automated. That this is so is proven by the construction and successful functioning of a machine which is able to receive a lyophilised sample of glycoconjugate and successfully deliver the intact O-glycans previously associated with that glycoconjugate.

## Claims

1. A method for isolating a glycan (as hereinbefore defined) which has been released from a glycoconjugate characterised in that the method includes sorbing the glycan (and any derivative thereof) upon a chromatographic material, separating the glycan from any remaining conjugate and/or conjugate-derived material, and recovering the glycan.

2. A method as claimed in Claim 1 for isolating a glycan from a post-release reaction mixture formed by the release of the glycan from a glycoconjugate which method includes contacting the post-release reaction mixture with a chromatographic material thereby to effect sorbing of the glycan (and any derivative thereof) upon the chromatographic material, eluting the chromatographic material, said sorbing and said eluting being such as to effect separation of glycan (and any derivative thereof) from conjugate and/or conjugate-derived material and such as to remove any releasing agent used in the release of glycan from the glycoconjugate, separating the glycan from any remaining conjugate or conjugate-derived material, and recovering the glycan.

3. A method as claimed in Claim 1 or Claim 2 for isolating a glycan from a post-release reaction mixture formed by the release of the glycan from a glycoconjugate which method includes contacting the post-release mixture with a first chromatographic material thereby to effect sorbing of the glycan (and any derivative thereof) upon the chromatographic material, eluting the chromatographic material, said sorbing and said eluting being such as to effect separation of glycan (and any derivative thereof) from conjugate and/or conjugate-derived material and such as to remove any releasing agent used in the release of glycan from the glycoconjugate, and, if a derivative of a glycan is present which has free primary amino acid groups, performing an N-acetylation reaction to generate unreduced glycan from any glycan derivative which has free primary amino acid groups, removing mono- and/or di-valent cations from a reaction mixture obtained after the N-acetylation reaction, if the manner in which the N-acetyl reaction was performed makes this desirable, separating from the reaction mixture glycan, or a derivative thereof, by means of sorption upon a second chromatographic material and selective elution therefrom, and converting any derivative of the glycan to unreduced glycan.

4. A method as claimed in Claim 3 wherein a glycan and any derivative thereof is eluted from the first chromatographic material prior to performing an N-acetylation reaction.

5. A method as claimed in Claim 3 wherein a glycan and any derivative thereof is subjected to an N-acetylation reaction prior to being eluted from the first chromatographic material.

6. A method as claimed in any one of Claims 1 to 5 wherein any acetohydrazone derivative of a glycan is subjected to acidic conditions to generate unreduced glycan.

7. A method as claimed in Claim 6 wherein acidic conditions are applied by means of acid in immobilised form, mineral acid, or a Lewis acid.

8. A method as claimed in any one of Claims 1 to 7 wherein the glycan is an N-glycan, an O-glycan, or N- and O-glycans.

9. A method as claimed in any one of Claims 1 to 8 wherein the glycoconjugate from which a glycan has been released is a glycoprotein, a glycohormone, a glycolipid or a mucin.

10. A method as claimed in any one of Claims 1 to 9 wherein an N-glycan is isolated after release from bovine fetuin, human serum $\alpha$1-acid glycoprotein, horseradish peroxidase or hen egg ovalbumin.

11. A method as claimed in any one of Claims 1 to 9 wherein an O-glycan is isolated after release from bovine fetuin, hog sub-maxilliary mucin or asialo globoside.

12. A method as claimed in any one of Claims 1 to 9 wherein N- and O-glycan are isolated after release from bovine fetuin.

13. A method as claimed in any one of Claims 1 to 12 wherein a microcrystalline cellulose or a $C_{18}$ reverse-phase material is used as a chromatographic material.

14. A method as claimed in any one of Claims 1 to 13 wherein a high yield of glycan is obtained.

15. A method as claimed in any one of Claims 1 to 14 wherein a glycan to be isolated has been released from a glycoconjugate by use of a releasing agent comprising an enzyme or a chemical reagent.

16. A method as claimed in Claim 15 wherein the chemical reagent is a hydrazine reagent.

FIG. 1

FIG. 2

EP 0 462 795 A2

**FIG. 3**

FIG. 4

RETENTION TIME (minutes)

FIG. 5

Retention time (minutes)

FIG. 6

FIG. 7

EP 0 462 795 A2

FIG. 8

Retention time (minutes)

**FIG. 9**

FIG. 10

EP 0 462 795 A2

FIG. 11

**FIG. 12**

# FIG. 13

EMO   Globoside (Sigma) TV2